Europäisches Patentamt

European Patent Office

Office européen des brevets

(19)

(11) **EP 0 830 126 B1**

(12) **FASCICULE DE BREVET EUROPEEN**

(45) Date de publication et mention
de la délivrance du brevet:
**25.07.2001 Bulletin 2001/30**

(21) Numéro de dépôt: **96917524.9**

(22) Date de dépôt: **21.05.1996**

(51) Int Cl.⁷: $A61K\ 7/00$, $A61K\ 9/107$

(86) Numéro de dépôt international:
**PCT/FR96/00756**

(87) Numéro de publication internationale:
**WO 96/37180 (28.11.1996 Gazette 1996/52)**

(54) **PSEUDO-EMULSIONS STABILISEES ET LEUR PROCEDE DE PREPARATION**

STABILISIERTE PSEUDOEMULSIONEN UND VERFAHREN ZU IHRER HERSTELLUNG

STABILIZED PSEUDO-EMULSIONS AND THEIR PREPARATION PROCESS

(84) Etats contractants désignés:
**AT BE CH DE DK ES FI FR GB GR IE IT LI LU MC NL PT SE**

(30) Priorité: **22.05.1995 FR 9506044**

(43) Date de publication de la demande:
**25.03.1998 Bulletin 1998/13**

(73) Titulaire: **Pierre Fabre Dermo-Cosmetique
92100 Boulogne (FR)**

(72) Inventeurs:
 • **TREBOSC, Marie-Thérèse
  F-81100 Castres (FR)**
 • **DUBOIS, Jacques
  F-11100 Narbonne (FR)**

(74) Mandataire: **Ahner, Francis
  Cabinet Régimbeau
  20, rue de Chazelles
  75847 Paris cedex 17 (FR)**

(56) Documents cités:
 **EP-A- 0 293 766      EP-A- 0 328 355
 DE-A- 4 425 268      FR-A- 2 710 263**

## Description

La présente invention concerne de nouvelles formulations de compositions galéniques hétérogènes, utilisables notamment en dermatologie et en cosmétologie.

Les formes galéniques hétérogènes constituent le degré ultime de complexité, en raison de la présence de composés à la fois lipophiles et hydrophiles : elles ont pour vocation de nettoyer, traiter, protéger et embellir la peau dans un souci de soin, de confort et d'agrément de l'utilisateur.

L'art de la galénique a toujours trouvé dans la réalisation des émulsions ses lettres de noblesse.

Cet art a été essentiellement constitué depuis toujours par la recherche et/ou la mise au point de substances émulsionnantes ou tensio actives pouvant mettre en suspension stable l'huile et l'eau réputées immiscibles.

Ces émulsionnants sont toujours d'origines diverses : synthétiques pour la plupart ou naturelles telles que les lécithines par exemple.

Quelles que soient leurs origines et la puissance de leur pouvoir émulsionnant le rôle d'un émulsionnant peut être rapproché de celui des détergents connus pour leur agressivité aussi bien sur les fibres textiles que sur les tissus cutanés.

Pour des raisons de fabrications industrielles et de stabilités physiques dans le temps, toutes les émulsions cosmétiques contiennent des émulsionnants qui peuvent être de nature non ionique (ex. : dérivés oxyéthylènés ou non de sorbitol, dérivés de sucrose), anioniques (sels d'acides gras) ou cationiques (dérivés d'ammonium quaternaires) les moins souvent utilisés cependant car classés parmi les plus agressifs et plus récemment des polymères émulsionnants connus sous le nom de Pemulen® .

Cependant, ces émulsionnants même choisis parmi les meilleurs restent redoutables pour la peau pour plusieurs raisons.

La peau est recouverte d'un film protecteur, appelé film hydrolipidique de surface (FHLS) et constitué en grande partie des corps gras excrétés par les glandes sébacées et des lipides provenant de la dégradation des cellules lors de la phase de kératinisation des cellules cornées.

A l'étalement sur la peau, l'émulsion est dissociée et la phase aqueuse externe s'évapore laissant en place l'émulsionnant qui présente une affinité pour les huiles ou corps gras du (FHLS) ; c'est ainsi qu'elle facilite l'élimination ultérieure de ce film de la surface de l'épiderme.

Ce film sera ainsi appauvri jour après jour. Seules les peaux grasses largement pourvues en glandes sébacées sécrétrices de sébum pourront compenser cette destruction. Mais on connaît cependant le phénomène de séborrhée réactionnelle occasionné par l'utilisation de tensioactifs trop agressifs pour le nettoyage quotidien.

Quant aux peaux sèches, elles sont déjà défavorisées par un FHLS déficient.

De plus, le ciment lipidique liant les cellules cornées est désorganisé et ne joue plus son rôle de barrière et sur certaines catégories de peaux fragilisées dites sensibles, personnes âgés, jeunes enfants atopiques, certaines réactions pourront plus facilement survenir.

On sait par ailleurs que l'appauvrissement de cet effet barrière augmente la fréquence des réactions irritatives dites orthoergiques et que l'application répétée de produits sur ce type de peau peut entraîner à la longue des réactions de sensibilisation à l'un des constituants - réactions dites allergiques.

Les émulsionnants agissent en outre comme des adjuvants de pénétration transcutanée ; à ce titre, ils révèlent l'effet irritant ou allergisant de diverses autres susbtances présentes dans la formulation qui serait parfaitement tolérée en leur absence.

Ils possèdent un effet cyto-toxique in vitro en culture cellulaire. Cette toxicité illustre l'action directe que peuvent excercer certains émulsionnants selon leur - nature et leur concentration - sur les cellules épidermiques dépourvues de leur protection naturelle fragilisée puis finalement lissées en raison des altérations progressives subies par la membrane cytoplasmique.

Toutes les émulsions actuelles (voir par exemple les documents EP-A-0328355, EP-A-0293766 et FR-A-2710263) contiennent des émulsifiants en application d'un principe fondamental de formulation définitivement admis ; le rôle du formulateur constitue donc à choisir un émulsionnant et à déterminer sa concentration. C'est dans ce contexte que les Demandeurs ont réalisé une observation surprenante et contraire à ce principe fondamental : il est possible dans certaines conditions d'obtenir des compositions hétérogènes pouvant être assimilées à des "pseudo-émulsions", en l'absence d'émulsionnants.

C'est pourquoi la présente invention a pour objet une composition pharmaceutique et/ou cosmétique sous forme de pseudo-émulsion,

caractérisée en ce qu'elle consiste essentiellement en

(1) au moins une phase aqueuse, contenant un seul agent gélifiant,

(2) au moins une phase lipidique contenant au moins un facteur de consistance,

$$\text{avec un rapport } \frac{\text{facteur de consistance}}{\text{phase lipidique totale}}$$

compris entre environ 0,06 et 0,18

et en ce que ladite composition est dépourvue de tensioactifs.

Les compositions selon l'invention sont particulièrement adaptés à un usage topique et jouent un rôle de protection ou de nettoyage avec un grand confort pour l'utilisateur, tout en supprimant les émulsionnants ainsi que les substances susceptibles d'être ou de devenir allergisantes ou irritantes.

De tels produits seront donc de très haute tolérance.

En effet les Demandeurs ont trouvé de manière inattendue que la suppression des émulsionnants était possible tout en conservant des émulsions stables dans le temps, en épaississant les phases antagonistes (hydrophiles et lipophiles) en présence. Les deux phases sont ainsi maintenues intimement liées durant de longues périodes, compatibles avec la vie d'un cosmétique, et cela pour différentes proportions de phases, ce qui permet d'envisager toute une gamme haute tolérance depuis des laits fluides jusqu'à des crèmes très riches et compactes.

On obtient des compositions hétérogènes, dont la structure microscopique est différente de celle d'une émulsion.

Le facteur de consistance présent dans la phase lipidique est une substance semi-solide à température ambiante et à point de fusion supérieur à 50° C ; solubilisé dans une phase grasse à chaud, il retrouve sa consistance semi-solide et confère à la phase lipidique à froid, une viscosité et une consistance qui sont réglés par le pourcentage de cette substance. Le rapport facteur de consistance/phase grasse totale définit la stabilité dans le temps du produit.

Ce rapport est, selon un aspect particulièrement préféré de l'invention, compris entre 0,08 et 0,15.

Les compositions selon l'invention présentent une bonne stabilité après 5 mois à 40° C, tant pour les formes fluides que pour les crèmes.

Ces facteurs de consistance sont choisis parmi des corps gras cireux tels que hydrocarbures, saturés, les triglycérides et les graisses végétales présentant un point de fusion supérieur à 50° C, les mono, diglycérides. Ces substances peuvent être d'origine animale, végétale ou synthétique. Le choix fait est celui synthétique qui permet une plus grande régularité dans les caractéristiques physico-chimiques notamment pour les mono ou diesters de glycérols pour lesquels le pourcentage relatif des mono ou diglycérides pourra varier de 40 à 100%. Les esters de glycérol utilisés en tant que facteur de consistance n'ont aucune action tensioactive : ils ont un HLB voisin de 3 et sont essentiellement lipophiles.

Un grand choix de composition de la phase grasse est rendu possible par les solubilités préférentielles de chacun des esters de glycérol dans un éventail très large d'huiles ou d'esters gras : par exemple, huiles végétales, huiles minérales, pérhydrosqualène, palmitate d'isopropyle, huiles de silicone conduisant à des phases huileuses plus ou moins riches, plus ou moins fluides, d'étalement, de pénétration et d'effet résiduel variés.

La phase aqueuse peut être épaissie par des gélifiants connus de l'homme du métier.

Ces substances sont destinées à donner de la consistance à la préparation en augmentant la viscosité de la phase aqueuse. Pour cet usage, des polymères sont employés, qui possèdent la propriété de former un réseau macromoléculaire en présence d'eau : un gel.

Les polymères peuvent être d'origine végétale : polyoses (polysaccharides) : gélose, gomme, en particulier les gommes xanthanes, cellulose, alginates, ou des dérivés semi-synthétiques, en particulier, les dérivés de la cellulose comme la méthylcellulose, l'éthylcellulose, l'hydroxypropylméthylcellulose qui sont des produits très utilisés en raison de leur souplesse d'emploi.

Ils peuvent également être de nature minérale : silices, silicates, bentones, ou encore synthétiques comme les polymères acryliques : carbopols (ou carbomers), qui sont des substances acides qu'il faut neutraliser au moment de l'emploi pour former le polymère : ce produit est donc toujours accompagné d'un alcalin (en général, une base organique telle que la tri-éthanolamine).

Parmi les polymères acryliques synthétiques, la qualité Synthalen® , (homopolymère de l'acide acrylique commercialisé par la société 3V-Sigma), dépourvue de résidus de synthèse tel que le benzène, est particulièrement adaptée.

Les compositions selon l'invention seront de préférence dépourvues de parfum.

Elles peuvent en outre contenir un composé actif par voie topique, soluble dans l'une et/ou l'autre des phases de l'émulsion, qui servira de véhicule pour le principe actif. Parmi les principes actifs susceptibles d'entrer dans les compositions selon l'invention. on peut citer ceux possédant des propriétés d'hydratant, émollient, kératoplastique, régénérateur, etc.

Les pseudo-émulsions selon l'invention sont particulièrement adaptées à la préparation de crèmes solaires, par incorporation d'un filtre ou d'un écran solaire.

Selon un mode de réalisation particulièrement avantageux, les conservateurs sont également absents des compositions selon l'invention.

Elles sont adaptées à une application sur la peau saine et aussi en raison de leur parfaite tolérance sur la peau lésée (abrasion superficielle, brûlures) et sur les muqueuses. L'absence de conservateurs constituant un atout supplémentaire pour de tels usages.

Cette parfaite tolérance a été démontrée par un test épicutané sous patch occlusif de 24 heures sur peau saine et sur peau lésée (par le lauryl ether sulfate à 0,5%).

L'évaluation de la tolérance cutanée est suivie par la mesure de la microcirculation au laser doppler.

On sait que les conservateurs (bien qu'autorisés) finissent par provoquer par leur emploi répété des réactions allergiques.

Les plus réputés dans ce cas sont les libérateurs de formol, la substance appelée Kathon CG (dérivés d'isothiazolone) et même les parabens (esters parahydroxybenzoïques).

Leur suppression pure et simple permet aux nombreux utilisateurs déjà sensibilisés tant par des spécialités cosmétiques que médicales (nombreux topiques contenant des parabens) de pouvoir utiliser ces produits sans crainte.

De plus, aucune autre substance ayant des propriétés bactériostatiques mais non listée dans la liste positive de la CEE n'est introduite ici, afin d'éviter de provoquer à la longue, de tels inconvénients.

Le même souci a fait supprimer les parfums ou toute substance introduite dans des compositions parfumantes susceptibles de masquer des odeurs.

Cette suppression impose en retour une rigueur particulière dans le choix des matières premières utilisées dans les formulations : leur nature, leur qualité et leur pureté chimique doivent être telles que leur caractère odorant, propre très faible ou nul.

La réalisation à l'échelle industrielle de ce type de mélange nécessite des procédés de fabrication très particuliers compliqués du fait de l'absence de conservateurs.

C'est pourquoi la présente invention à également pour objet un procédé de préparation d'une composition sous forme d'émulsion dépourvue d'agents tensio-actifs, caractérisé en ce qu'on effectue, en atmosphère stérile, les étapes suivantes :

a) préparation des phases respectivement aqueuse et lipidique, à une température supérieure ou égale à environ 70° C,
b) introduction, sous agitation élevée, de la phase lipidique dans la phase aqueuse, en maintenant la température supérieure ou égale à environ 70° C,
c) refroidissement du mélange jusqu'à une température supérieure ou égale à environ d'environ 25° C, sous agitation modérée,
d) récupération d'une émulsion lisse stérile.

L'aspect définitif du produit n'est atteint qu'à la température de 25° C où les deux phases finissent par être parfaitement lissées.

Dans ce procédé toute substance solide doit être préalablement stérilisée et introduite soit directement par le vide dans le réacteur (selon la technique du lit fluidisé), soit préalablement dissoute dans une solution qui devra subir une filtration stérilisante préalable.

Chacune des phases est maintenue à une température supérieure à 70° pendant toute la durée du mélange, de la filtration et du transfert dans la cuve de fabrication, par des systèmes de calorifugeage.

Le procédé de fabrication est original dans le sens où la phase grasse maintenue à 70° est introduite sous agitation élevée type Turbine durant un temps très court - 5 à 10 mn dans la phase aqueuse légèrement gélifiée par une neutralisation partielle ; la vitesse d'agitation sera avantageusement comprise entre 20 et 30 m/s. La fin de la neutralisation s'effectue ensuite avant refroidissement sous agitation modérée ; celle-ci peut plus particulièrement être obtenue par des systèmes à Hélice, ancre, à des vitesses inférieures ou égales à environ 2 m/s.

Ensuite, le stockage et la répartition en conditionnements eux-mêmes stériles assurent la parfaite protection.

Les exemples qui suivent sont destinés à illustrer l'invention.

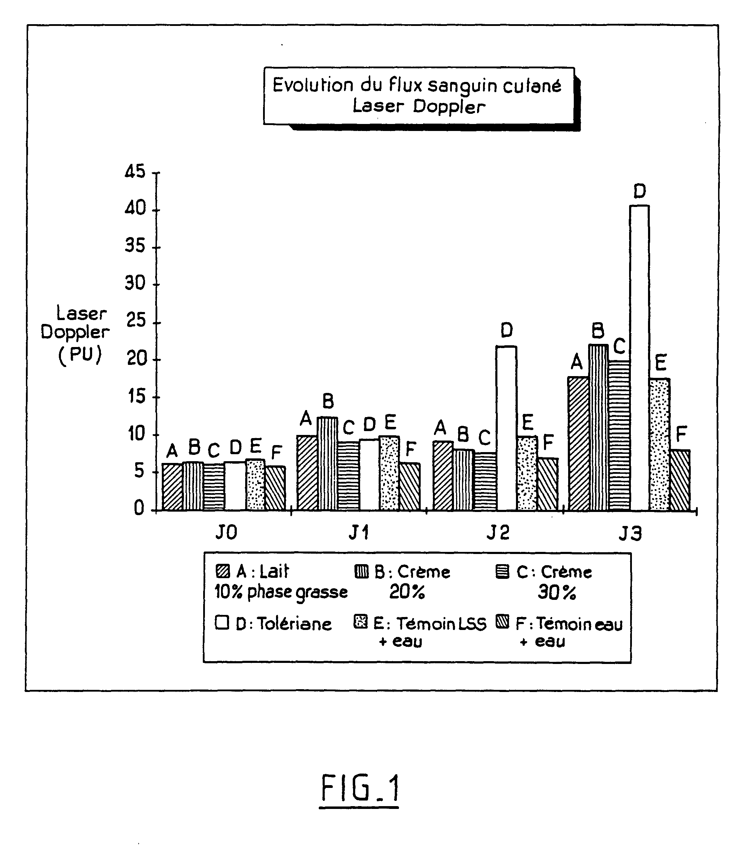

Dans ces exemples, on se réfèrera à la figure en annexe, qui représente l'évolution du flux sanguin cutané après application de compositions sous patch occlusifs.

**Exemple 1 : lait démaquillant**

Phase aqueuse :        90%

. eau déminéralisée ou thermale        79 à 75 g
. glycérine        11 à 15 g
. polymère carboxyvinylique :        0,3% (sans trace de benzène)
. triéthanolamine extra pure        q.s.p. pH 6

Phase grasse :        10%

. paraffine liquide        8,5 g
. monostéarate de glycérol :        1,5 g

**Exemple 2 : crème pour peaux sèches et sensibles**

Phase aqueuse :        80%

. eau déminéralisée        69 à 65 g
. glycérine        11 à 15 g
. polymère carboxyvinylique :        0,25% (sans benzène)
. TEA extra pure        q.s.p. pH 6

Phase grasse :        20%

. monostéarate de glycérol (40%)        2,4 g
. huile minérale        5 g
. huile de silicone        2 g
. huile végétale        6,6 g
. pérhydrosqualène        5 g
       A cette phase peut être ajoutée la vitamine E naturelle ou la forme acétate à 0,5% maximum comme protecteur contre l'oxydation.

Autre exemple de la phase grasse : 20%

- monostéarate de glycérol à 100% de monoester 2,5 g
- cyclométhicone 6 g
- cosbiol 7,5 g
- huile de sésame 4 g

## Exemple 3 : crème pour peaux délipidées ou atopiques

Phase aqueuse : 70%

- eau déminéralisée ou thermale 59 à 55 g
- glycérol 11 à 15 g
- polymère carboxyvinylique : 0,2%

Phase grasse : 30%

- monostéarate de glycérol : 2,6 g
- huile végétale : 15,4 g
- pérhydrosqualène : 8 g
- paraffine fluide : 4 g

## Exemple 4 : crème solaire écran total

A l'exemple précédent 4 à 8% de dioxyde de Titane micronisé et préalablement stérilisé peut être dispersé dans la phase grasse.

## Exemple 5 : Evaluation de la tolérance cutanée

L'étude est réalisée par mesure, au laser doppler de la microcirculation cutanée après agression au Lauryl sulfate sodique (LSS) puis application des produits sous patches occlusifs pendant 24 h.

Les mesures sont effectuées :

- à JO : avant toute application du produit.
- à J1 : 1 h après retrait du patch au LSS.
- à J2 : 1 h après retrait des patches produits et du patch témoin eau.
- à J3 : 24 h après retrait des patches produits et du patch témoin eau.

Les résultats sont représentés sur la figure en annexe.

Cette étude comportait un référent du marché : Tolériane présenté comme un produit de haute tolérance, sans conservateur.

Les essais effectués avec les produits A, B et C correspondant respectivement aux exemples 1, 2 et 3 sont beaucoup mieux tolérés que le produit de référence.

L'augmentation de la microcirculation, témoin d'une inflammation locale liée à une irritation, est du même ordre que celle observée avec le témoin LSS + eau.

Cette augmentation est significativement inférieure à l'évolution du produit de référence.

## Revendications

1. Composition pharmaceutique et/ou cosmétique sous forme de pseudo-émulsion stabilisée, caractérisée en ce qu'elle consiste essentiellement en

   (1) au moins une phase aqueuse, contenant un seul agent gélifiant, choisi dans le groupe comprenant les polyoses tels que la gélose, les gommes xanthanes, la cellulose, les alginates, les dérivés hémisynthétiques de la cellulose et les polymères acryliques tel le Synthalen® .
   (2) au moins une phase lipidique contenant au moins un facteur de consistance,

   avec un rapport $\dfrac{\text{facteur de consistance}}{\text{phase lipidique totale}}$ compris entre environ 0,06 et 0,18
   et en ce que ladite composition est dépourvue de tensioactifs.

2. Composition selon la revendication 1, caractérisée en ce que le rapport facteur de consistance / phase lipidique totale est compris entre 0,08 et 0,15.

3. Composition selon l'une des revendications 1 ou 2, caractérisée en ce que le facteur de consistance présent dans la phase lipidique est choisi dans le groupe comprenant les cires, les hydrocarbures saturés, les monoglycérides, les diglycérides, les triglycérides et les graisses végétales présentant un point de fusion supérieur à environ 50° C.

4. Composition selon l'une des revendications 1 à 3, caractérisée en ce qu'elle contient en outre au moins un principe actif, dans au moins une des phases de la pseudo-émulsion stabilisée.

5. Procédé de préparation d'une composition sous forme de pseudo-émulsion stabilisée selon l'une des revendications 1 à 4, caractérisé en ce qu'on effectue, en atmosphère stérile, les étapes suivantes :

   a) préparation des phases respectivement aqueuse et lipidique, à une température supérieure ou égale à environ 70° C,
   b) introduction, sous agitation élevée, de la phase lipidique dans la phase aqueuse, en maintenant la température supérieure ou égale à environ 70° C,
   c) refroidissement du mélange jusqu'à une température d'environ 25° C, sous agitation modérée,
   d) récupération d'une émulsion lisse stérile.

**6.** Composition susceptible d'être obtenue par le procédé selon la revendication 5, caractérisée en ce qu'elle est dépourvue de tensioactifs et de conservateurs.

**7.** Composition selon la revendication 6, caractérisée en ce qu'elle présente la formule suivante :

Phase aqueuse :    90%

- eau déminéralisée ou thermale    79 à 75 g
- glycérine    11 à 15 g
- polymère carboxyvinylique :    0,3% (sans trace de benzène)
- triéthanolamine extra pure    q.s.p. pH 6

Phase grasse :    10%

- paraffine liquide    8,5 g
- monostéarate de glycérol :    1,5 g

**8.** Composition selon la revendication 6, caractérisée en ce qu'elle présente la formule suivante :

Phase aqueuse :    80%

- eau déminéralisée    69 à 65 g
- glycérine    11 à 15 g
- polymère carboxyvinylique :    0,25% (sans benzène)
- TEA extra pure    q.s.p. pH 6

Phase grasse :    20%

- monostéarate de glycérol (40%)    2,4 g
- huile minérale    5 g
- huile de silicone    2 g
- huile végétale    6,6 g
- pérhydrosqualène    5 g

**9.** Composition selon la revendication 6, caractérisée en ce qu'elle présente la formule suivante :

Phase aqueuse :    70%

- eau déminéralisée ou thermale    59 à 55 g
- glycérol    11 à 15 g
- polymère carboxyvinylique :    0,2%

Phase grasse :    30%

- monostéarate de glycérol :    2,6 g
- huile végétale :    15,4 g
- pérhydrosqualène :    8 g
- paraffine fluide :    4 g

**10.** Composition selon la revendication 9, caractérisée en ce qu'elle contient en outre 4 à 8% en poids de dioxyde de titane.

**11.** Composition selon l'une des revendications 6 à 8, caractérisée en ce qu'elle contient en outre un agent actif par voie topique.

**Claims**

**1.** Pharmaceutical and/or cosmetic composition in the form of stabilized pseudoemulsion, characterized in that it consists essentially of

(1) at least one aqueous phase containing a single gelling agent chosen from the group including polyoses such as agar, xanthan gums, cellulose, alginates, semisynthetic derivatives of cellulose and acrylic polymers such as Synthalen® .
(2) at least one lipid phase containing at least one consistency factor,

with a consistency factor/total lipid phase ratio of between approximately 0.06 and 0.18 and in that the said composition is devoid of surfactants.

**2.** Composition according to Claim 1, characterized in that the consistency factor/total lipid phase ratio is between 0.08 and 0.15.

**3.** Composition according to either of Claims 1 and 2, characterized in that the consistency factor present in the lipid phase is chosen from the group including waxes, saturated hydrocarbons, monoglycerides, diglycerides, triglycerides and vegetable fats which have a melting point higher than approximately 50°C.

**4.** Composition according to one of Claims 1 to 3, characterized in that it additionally contains at least one active principle, in at least one of the phases of the stabilized pseudoemulsion.

**5.** Process for the preparation of a composition in the form of stabilized pseudoemulsion according to one of Claims 1 to 4, characterized in that the following steps are performed, in sterile atmosphere:

a) preparation of the aqueous and lipid phases respectively, at a temperature higher than or equal to approximately 70°C,
b) introduction, with high stirring, of the lipid phase into the aqueous phase, while maintaining the temperature higher than or equal to approximately 70°C,

c) cooling the mixture to a temperature of approximately 25°C, with moderate stirring,
d) recovery of a sterile smooth emulsion.

**6.** Composition capable of being obtained by the process according to Claim 5, characterized in that it is devoid of surfactants and of preserving agents.

**7.** Composition according to Claim 6, characterized in that it has the following formulation:

Aqueous phase: 90 %

- demineralized or thermal water     79 to 75 g
- glycerine     11 to 15 g
- carboxyvinyl polymer:     0.3 % (without trace of benzene)
- extra pure triethanolamine     q.s. pH 6

Fatty phase: 10 %

- liquid paraffin     8.5 g
- glycerol monostearate:     1.5 g

**8.** Composition according to Claim 6, characterized in that it has the following formulation:

Aqueous phase: 80 %

- demineralized water     69 to 65 g
- glycerine     11 to 15 g
- carboxyvinyl polymer:     0.25 % (without benzene)
- extra pure TEA     q.s. pH 6

Fatty phase: 20 %

- glycerol monostearate (40 %)     2.4 g
- mineral oil     5 g
- silicone oil     2 g
- vegetable oil     6.6 g
- perhydrosqualene     5 g

**9.** Composition according to Claim 6, characterized in that it has the following formulation:

Aqueous phase: 70 %

- demineralized or thermal water     59 to 55 g
- glycerol     11 to 15 g
- carboxyvinyl polymer:     0.2 %

Fatty phase: 30 %

- glycerol monostearate:     2.6 g
- vegetable oil:     15.4 g

- perhydrosqualene:     8 g
- fluid paraffin:     4 g

**10.** Composition according to Claim 9, characterized in that it additionally contains 4 to 8 % by weight of titanium dioxide.

**11.** Composition according to one of Claims 6 to 8, characterized in that it additionally contains an agent which is active by topical route.

**Patentansprüche**

**1.** Pharmazeutische und/oder kosmetische Zusammensetzung in Form einer stabilisierten Pseudoemulsion, dadurch gekennzeichnet, dass sie im wesentlichen besteht aus

(1) mindestens einer wässrigen Phase, die ein einziges Geliermittel enthält, das aus der Gruppe ausgewählt ist, die Polyosen, wie Agar-Agar, Xanthangummis, Cellulose, Alginate, halbsynthetische Derivate von Cellulose, und acrylische Polymere, wie Synthalen® , umfasst;
(2) mindestens einer Lipidphase, die mindestens einen Konsistenzfaktor enthält,

mit einem Verhältnis $\dfrac{\text{Konsistenzfaktor}}{\text{Gesamt-Lipidphase}}$ das zwischen etwa 0,06 und 0,18 eingeschossen ist, und dass die Zusammensetzung kein Tensid enthält.

**2.** Zusammensetzung nach Anspruch 1, dadurch gekennzeichnet, dass das Verhältnis Konsistenzfaktor/Gesamt-Lipidphase zwischen 0,08 und 0,15 eingeschlossen ist.

**3.** Zusammensetzung nach einem der Ansprüche 1 oder 2, dadurch gekennzeichnet, dass der Konsistenzfaktor, der in der Lipidphase vorliegt, aus der Gruppe ausgewählt ist, die Wachse, gesättigte Kohlenwasserstoffe, Monoglyceride, Diglyceride, Triglyceride und Pflanzenfette, welche einen Schmelzpunkt oberhalb von etwa 50°C aufweisen, umfasst.

**4.** Zusammensetzung nach einem der Ansprüche 1 bis 3, dadurch gekennzeichnet, dass sie darüber hinaus mindestens einen Wirkstoff in mindestens einer der Phasen der stabilisierten Pseudoemulsion enthält.

**5.** Verfahren zur Herstellung einer Zusammensetzung in Form einer stabilisierten Pseudoemulsion nach einem der Ansprüche 1 bis 4, dadurch gekennzeichnet, dass man in steriler Atmosphäre die folgenden Schritte durchführt:

(a) Herstellung der wässrigen bzw. Lipidphase bei einer Temperatur oberhalb von oder gleich etwa 70°C,
(b) Einführen der Lipidphase in die wässrige Phase unter verstärktem Rühren, wobei man die Temperatur oberhalb von oder gleich etwa 70°C hält,
(c) Abkühlen der Mischung bis auf eine Temperatur von etwa 25°C unter mäßigem Rühren,
(d) Gewinnen einer gleichförmigen sterilen Emulsion.

**6.** Zusammensetzung, die geeignet ist, durch das Verfahren gemäß Anspruch 5 erhalten zu werden, dadurch gekennzeichnet, dass sie kein Tensid und keine Konservierungsmittel enthält.

**7.** Zusammensetzung nach Anspruch 6, dadurch gekennzeichnet, dass sie die folgende Formulierung aufweist:

Wässrige Phase:        90 %

- demineralisiertes oder Thermalquellen-Wasser        79 bis 75 g
- Glycerin        11 bis 15 g
- carboxyvinylisches Polymer:        0,3 % (ohne Spur von Benzol)
- extra reines Triethanolamin        q.s.p. pH 6

Fettphase:        10 %

- flüssiges Paraffin        8,5 g
- Glycerinmonostearat:        1,5 g

**8.** Zusammensetzung nach Anspruch 6, dadurch gekennzeichnet, dass sie die folgende Formulierung aufweist :

Wässrige Phase:        80 %

- demineralisiertes Wasser        69 bis 65 g
- Glycerin        11 bis 15 g
- carboxyvinylisches Polymer:        0,25 % (ohne Benzol)
- extra reines TEA        q.s.p. pH 6

Fettphase:        20 %

- Glycerinmonostearat (40 %)        2,4 g
- Mineralöl        5 g
- Siliconöl        2 g
- Pflanzenöl        6,6 g

- Perhydrosqualen        5 g

**9.** Zusammensetzung nach Anspruch 6, dadurch gekennzeichnet, daß sie die folgende Formulierung aufweist:

Wässrige Phase:        70 %

- demineralisiertes oder Thermalquellen-Wasser        59 bis 55 g
- Glycerin        11 bis 15 g
- carboxyvinylisches Polymer:        0,2 %

Fettphase:        30 %

- Glycerinmonostearat:        2,6 g
- Pflanzenöl:        15,4 g
- Perhydrosqualen:        8 g
- flüssiges Paraffin:        4 g

**10.** Zusammensetzung nach Anspruch 9, dadurch gekennzeichnet, dass sie darüber hinaus 4 bis 8 Gew.-% Titandioxid enthält.

**11.** Zusammensetzung nach einem der Ansprüche 6 bis 8, dadurch gekennzeichnet, dass sie darüber hinaus einen Wirkstoff enthält, der auf topischem Weg aktiv ist.

Evolution du flux sanguin cutané
Laser Doppler

FIG.1